# EUROPEAN PATENT APPLICATION

(11) **EP 2 704 042 A1**
(43) Date of publication of application: **05.03.2014**
(21) Application number: 12182583.0
(22) Date of filing: 31.08.2012
(51) Int. Cl.: G06F 19/00

(54) **Method for a medication administration system and a medication administration system**

(71) Applicant: Evondos Oy, 24240 Salo (FI)
(72) Inventor: Niinistö, Jyrki, 24800 Halikko (FI); Apell, Mika, 20700 Turku (FI)
(74) Representative: Mäkelä, Katja

(57) **Abstract**

The present invention relates to a method for a medication administration system, which system comprises a medication dispenser and a medication administration server. The method comprises sending a message to the system for assisting and instructing the patient remotely in medication or other care events and/or supplying a message to the system from the medication dispenser.

## Description

### TECHNICAL FIELD OF THE INVENTION

The present invention relates to a method for a medication administration system and to a medication administration system according to the preambles of the appended independent claims.

### BACKGROUND OF THE INVENTION

Many medical conditions require long-term or permanent medication. A patient must usually take one or more medications in prescribed dosages and at certain time intervals. However, taking medicines according to medical regimen is not trivial, especially for elderly who typically have more than one pill to be taken more than once a day. This also concerns other self-care events. Medical care is often the first reason why elderly people need assistance from caregivers, public healthcare or close relatives. Caregivers visit patients often to dispense medications and to make sure that medications are taken correctly according to medical regimen. They can also monitor a patient's condition during the visits. Caregivers use a lot of time for travelling and this time is out of actual care giving tasks. Medication administration is often the first reason why patients need to leave their homes from independent to assisted living.

Various devices are known for assisting the patient in complying with his/her medical regimen. The most sophisticated devices are so-called medication dispensers, which dispense pre-packaged and labelled medication packages to provide the patient with the proper dosage of medications at a prescribed time. The medications are pre-packaged into packages according to the medical regimen of the patient, and are available from licensed pharmacies. The labels of the medication packages may contain information about the patient, the content of the package, and the date and time of the dosage to be taken. The information may be, for example, in a form of text, a one- or two-dimensional bar code, an RFID (radio frequency identification) or an NFC (near field communication) tag, or a magnetic tag.

Typically, the medication packages are arranged as a strip, which is inserted into a container of the medication dispenser either by the patient or a caregiver of the patient. The medication dispenser dispenses the packages by separating the packages from the strip one package at a time according to the information provided by the labels of the packages, or information stored in the medication dispenser. Also other formats of medication packages e.g. blister packages or separate containers are known in the art and can be used. The medication dispenser allows the dispensation of medications to be monitored and controlled so that the patient, the caregiver or any other person having access to the apparatus can be assured that the patient is taking the medications as prescribed. Automated medication dispensers with monitoring features are known in the art, where the dispenser sends data to a server if the medications have been taken or not.

If medications are not taken correctly according to a medical regimen of the patient, their effect is not as planned by the physician, e.g. missing medications reduce the effect of medical care, taking more medications than prescribed or taking medications against medical regimen or medication specific instructions may cause harmful side effects. The medical care can also comprise some other care events which are critical relating to the effect of medical care. It is hard for the caregiver or the physician to control and to estimate if the medications have been taken correctly or not, and if the continued symptoms or side-effects are caused by this non-compliance or non-suitable or ineffective medications. This can lead to unnecessary change of medication or even new medications to new symptoms.

### OBJECTIVES OF THE INVENTION

It is the main objective of the present invention to provide a medication administration system which reduces or even eliminates problems presented above.

It is an objective of the present invention to provide a method for automated medication administration system, which assists and instructs a patient to take his/her medication correctly at the right time according to the patient's medical regimen and also to assist in other care events relating to the medical regimen and so to support independent living at home.

It is a further objective of the invention to provide remote messaging functions in a medication dispenser system for assisted care and also functions for a patient's condition follow-up.

In order to realise the above-mentioned objectives, the method and the medication dispenser according to the invention are characterised by what is presented in the characterising parts of the appended independent claims. Advantageous embodiments of the invention are described in the dependent claims.

### DESCRIPTION OF THE INVENTION

A typical method according to the present invention for a medication administration system, which system comprises a medication dispenser, which includes medication packages containing medications to be taken at predefined taking times, and a medication administration server which is arranged to be communicatively connected with the medication dispenser over a communications network, comprises:
- providing the medication dispenser with a patient profile, which profile comprises identification information of the patient and information related to medications of the patient and taking periods for the medication packages,
- providing the system with communication means, which are configured to receive and/or transmit a message, and
the method further comprises
- sending a message to the system for assisting and instructing the patient remotely in medication or other care events, linking the message to a predefined information in the patient profile, and notifying the patient of the received message,
and/or
- supplying a message to the system from the medication dispenser.

A typical medication administration system according to the present invention comprises
- a medication dispenser, which includes medication packages containing medications to be taken at predefined taking times,
- a medication administration server, which is arranged to be communicatively connected with the medication dispenser over a communications network,
- means for receiving a patient profile, which profile comprises identification information of the patient and information related to medications of the patient and taking periods for the medication packages,
- means for receiving a message for assisting and instructing the patient remotely in medication or other care events, and means for linking the message to a predefined information in the patient profile,
- means for notifying the patient of the received message, and
- means for supplying a message to the system from the medication dispenser.

A patient can be assisted and instructed remotely to take his/her medication correctly by a method and a system according to the invention. Instructions relating to the medication can be given remotely and they can be linked to medications of the patient. The patient can also be assisted and reminded of other care events relating to his/her medication in order for all care events to be carried out and the care to be effective and desired results will be achieved.

In an embodiment of the invention a direct feedback can be requested and received regularly e.g. about care effectiveness, symptoms or just feelings from the patient. Requested feedback can be related to physical or mental health or welfare. This helps especially if new medications are prescribed to the patient and effectiveness or side-effects of the medications are uncertain. In other words, caregivers, nurses or other predefined parties can remotely monitor a patient's condition by a method and a system according to the invention. In addition, the feedback from the patient provided by the method according to the invention gives important information to physicians to optimize the medication, or the system can even be used as a tool for medicine developers to develop better medicines more efficiently.

According to an embodiment of the invention the patient can ask for real-time help from predefined parties by using the medication administration system of the invention. Also, caregivers and other predefined parties can monitor the patient's condition and the status and the effect of self-care in real-time by using the system of the invention.

According to an embodiment of the invention the medication administration system can be used for recording and monitoring care events carried out in a location of the dispenser, i.e. typically at the home of the patient. Thus the system of the invention also provides a useful tool for caregivers for controlling that all necessary care events have been performed. The records can be saved e.g. in a memory of the dispenser, whereby the stored records of the care events, i.e. so-called log of the events, can be monitored afterwards by the caregiver or any other predefined party. These records can also be sent forward to the server to be monitored by predefined parties through web user interface from a terminal communicatively connected to the server. Typically, this care event information is linked to the patient profile defined in the system, at least to the identification information of the patient.

The system of the present invention enables communication between a predefined party and the user of the dispenser, i.e. typically the patient, either only in one direction or in both directions. Messages can be sent to the dispenser for assisting the patient in medication or care events, and/or messages can be sent from the dispenser to the predefined recipient for requesting help or for providing a required feedback. In a preferred embodiment of the invention, the messages can be sent in both directions, whereby the system of the invention provides an interactive tool for communication between the patient and the predefined parties.

The predefined recipient or party can be e.g. a caregiver, close relative, nurse, physician, or any other stakeholder. In an embodiment of the invention the dispenser can comprise multiple predefined parties which can send messages to the system or multiple recipients for messages which are sent from the dispenser. In an embodiment of the invention the recipient of the message is determined in the patient profile that is available in the system, in which profile the message is typically linked before it is sent forward to the recipient. An authorized user, typically an administrator, can define messaging contacts in the system. A messaging contact can be a predefined terminal such as a mobile device, e.g. a mobile phone or laptop, or another computer communicatively connected to the system. Messaging contacts can be defined e.g. with mobile device IDs, such as mobile device number. The administrator can represent the healthcare organization taking care of the patient, be a relative of the patient or the patient him/herself. Predefined messaging contacts can be available on the dispenser as predefined contacts and the system only permits messages from them. The purpose of predetermining messaging contacts is to protect the patient from inappropriate messages or connections to the dispenser of the patient and on the other hand to preconfigure the necessary message recipients to be easily available for the patient in the dispenser of the patient.

A medication administration system of the invention comprises a medication dispenser for medication packages containing medications to be taken at a predefined time based on information available on the packages or in the system. For remote monitoring and assistance the system of the invention comprises a medication administration server. The system of the invention comprises a patient profile which comprises identification information of the patient and information related to the medications of the patient. The information related to medications of the patient comprises e.g. medical regimen, dosages of medications and taking periods for the medication packages. In addition, the patient profile can comprise information about other predefined care events. In an embodiment of the invention the patient profile is stored in a database, which comprises information, i.e. records, relating to the patient and medications of the patient. The messages supplied to the system according to the invention can be linked to these records being included in the profile. Typically all information relating to the patient profile is stored in the server, from which it is provided to the medication dispenser, but it may also be stored in the local memory unit of the medication dispenser. In addition, the system of the invention comprises means for receiving a message sent to the system for assisting a patient remotely in medication or other care events, means for linking the message to a certain information in the patient profile, means for notifying the patient of the received message, and/or means for supplying a message to the system by using the medication dispenser. In a preferred embodiment of the invention the system comprises both means for receiving messages and means for transmitting messages. In a preferred embodiment the system further comprises means for sending a message from the medication dispenser to a server or a predefined recipient's terminal, e.g. to the recipient's mobile device.

A typical medication dispenser according to the system of the invention comprises a container arranged to receive a strip comprising packages of medication, each package having a label containing information relating to the package, a reader arranged to read information on a label of a package, transfer means for transferring packages from the container to the reader and then to an outlet of the medication dispenser, a control unit arranged to control the reader and the transfer means, and a communication unit arranged to receive and transmit messages. In a typical medication dispenser according to the invention the control unit is arranged to compare the information read from the label of the next package to be delivered from the dispenser with the information in the patient profile stored in the system in order to determine if the package can be dispensed to the patient and also to determine if the system comprises a message linked to the information of the package. The control unit is connected to the reader and the transfer means, and arranged to control the reader and the transfer means so that one package is dispensed at a time at determined times. The control unit comprises a processor that is programmed to carry out the functions that are needed to operate the medication dispenser. The control unit also comprises a memory for storing, for example, the patient information and the messages sent to the dispenser and messages supplied to the system from the dispenser.

The medication dispenser is arranged to dispense packages to provide the patient with the proper dosage of medications at a prescribed time. The time at which the packages are to be delivered to the patient is read either from the labels of the packages or from the patient profile available in the system. The medication dispenser allows the dispensation of medication to be monitored and controlled so that the patient, a caregiver of the patient or any other person having access to the medication dispenser can be assured that the patient is taking the medication as prescribed.

The medication dispenser of the system of the invention comprises user interface means for receiving the message from a server or a predefined recipient and means for notifying the patient of the received message.

According to the invention different instructive messages can be linked to information included in the patient profile available in the system. Linking to the information to the patient profile means that messages can be linked to different medications or other care events or to the identification information of the patient defined in the patient profile. Since in an embodiment of the invention the information of the packages to be dispensed is compared to the information in the patient profile before the package is delivered to the patient, the message sent to the system is also linked to the information of the medication packages, i.e. messages may be linked to the dispensing process of the packages (i.e. so-called medication event) and then the message creates a new instruction or care event relating to the patient's treatment. Messages may comprise a different kind of additional information relating to the medication and the treatment of the patient. Messages can be supplied to the system at any time and notified to the patient directly or only at predefined time. Messages can typically be modified by the creator until they have been notified to the patient. The message may comprise a time tagging which is dependent on e.g. the dispensing process of the medication packages for notifying the messages to the patient at a predefined time. The message sent to the system can include e.g. instructions relating to the required operations carried out with the medications, which are notified simultaneously with the dispensing process, e.g. "Drink one glass of water with the medication". Alternatively, the message can include instructions relating to the operations before and/or after medication intake, e.g. "8:00 medication has to be taken after breakfast". The message can also comprise instructions relating to the other care events which are required with the medication or during a certain time interval from the medication, e.g. "Measure blood pressure".

Typically, a message according to the invention comprises a text, which includes instructive information relating to the medication or other care events for assisting the patient in his/her care events. Alternatively, the message sent to the system may comprise only a data string which is compared to a library of the instructive messages available in the system and the instructive message which corresponds to the data string is selected from the library and linked to the patient profile. This data string can be alternatively included in the package information and read by the dispenser from the medication package.

In a method of the invention a message is sent to the server or directly to the dispenser, and so the linking of the message to the patient profile is carried out in the server or in the dispenser. The nurse or caregiver who sends messages to the system is typically responsible for linking the messages to the right events or packages and for timing of them.

In a method of the invention messages to the dispenser are sent by communication means via the server of the system or directly from a predefined terminal. When messages are sent to the dispenser directly from a stakeholder's mobile device, a phone number of the stakeholder needs to be predefined to the dispenser for security reasons.

In an embodiment of the invention, messages are sent to the system through web user interface from a terminal communicatively connected to the server for a specific patient.

In a method of the invention the message sent to the system is stored in a memory of the server or the dispenser. In a method of the invention the message can be notified to the patient immediately when it is sent to the system, or it can be notified when the medication package is dispensed from the dispenser, or notification can be timed to any other time. In an embodiment of the invention, the message is marked as read and logged as read in the memory of the dispenser or the server, when the message has been notified to the patient and he/she has acknowledged it.

In an embodiment of the invention the notification of the message is linked to the dispensing process of the medication package, in which method information from the medication package to be dispensed next from the dispenser is read, the information read from the package is compared to the information of the patient's profile stored in the system, and the received message linked to the patient profile and corresponding to the information read from the package is identified and retrieved from a memory of the server or the dispenser and notified to the patient.

In an embodiment of the invention the method comprises an audio and/or visual notification of the message in order to draw attention of the patient to the instructive message. The patient may be notified with an alarm that is played using a loudspeaker. The patient may be notified visually using a light source, such as a lamp or LED, or a display.

In a preferred embodiment of the invention the method comprises displaying the message on the user interface of the medication dispenser, i.e. the dispenser comprises a user interface for displaying messages such as instructions or other messages. The user interface means of the dispenser preferably comprises a touch screen, for example, a 7" touch screen. The large-sized touch screen enables a sufficiently big font for letters of instructions. Messages can also include other multimedia like photos, pictures, recorded audio or video which can be shown and/or played to the patient when reading the message.

In a method of the invention a message can be supplied to the system of the invention by using the medication dispenser. Typically, the messages supplied to the system are also linked to the identification information of the patient and/or medication information or care events defined in the patient profile, whereby it is possible to send the message forward to the predefined recipients.

In an embodiment of the invention a message is supplied to the dispenser by using user interface means of the dispenser and the message is stored in the memory of the dispenser or the server. This log of the system can be monitored afterwards.

The method of the invention can also comprise sending a message from the dispenser to a server or directly to a predefined recipient or party, e.g. to a predefined terminal such as a mobile device. According to an embodiment of the invention the control unit of the dispenser is arranged to send a message over a communications network to a server or to a predefined recipient. The recipient of the message is clearly defined to the system. There can be multiple predefined recipients depending on the required assistance of the user of the dispenser, e.g. for asking for additional information about medication the recipient can be a pharmaceutical information center or pharmacy. For requesting assistance to the care events the recipient can be caregiver or nurse.

In an embodiment of the invention the method comprises requesting feedback from the patient and transmitting the answer to a server or a predefined recipient, e.g. to the recipient's mobile device or other terminal. Typically, the message with feedback request comprises question and reply alternatives relating to the patient's physical or mental health or welfare displayed on the user interface of the dispenser, preferably on the touch screen. In a preferred embodiment of the invention answer alternatives of the feedback questions about the patient's condition are predefined, like "good", "bad", "neutral". Predefined questions and feedback can be selected by the predefined party when creating the message. Alternatively, question and feedback answers can be freely written by the predefined party but length of the texts may be limited to a certain amount of letters. In an embodiment of the invention a view on the local touch screen can be previewed through web user interface from a terminal communicatively connected to the server to ensure a clear message view.

In an embodiment of the invention a message sent from the dispenser, e.g. feedback, is stored in the log of the dispenser or the message is sent over a communications network to a server and it is stored in the server of the system. Thus messages and feedback can be monitored by the predefined party. Alternatively, feedback can be sent directly over a communications network to a predefined terminal, e.g. a caregiver's predefined mobile device. In a preferred embodiment of the invention all the information, which is entered to the system locally, e.g. by the user interface of the medication dispenser is transferred to the server and stored in the memory of the server.

In an embodiment of the invention the method comprises opening a voice or video call between the patient and a predefined recipient. In that case the dispenser comprises a microphone and speaker for a voice call and/or a camera for a video call. The voice call can be delivered as a VoIP call or a cellular mobile call. In a preferred embodiment of the invention a voice or video call can be opened to/from the dispenser for assistance if the patient needs information about a specific medication or assistance in care. The voice or video call can be opened to a predefined recipient. Alternatively, the caregiver can open connection to the dispenser to monitor taking medication or condition of the patient or giving additional instructions to the patient in real-time.

In an embodiment of the invention the system can send a photo of medicines to be taken, accompanied with their names, to the predefined recipient for giving care event related information.

In an embodiment of the invention access to a list of messages, messages with feedback questions and answers to feedback questions logged in the system is preferably restricted to the message creator and the patient. Only authorized users like administrators may have access to all messages.

As disclosed above, the system and the method of the invention provide a useful tool for the caregivers for giving information relating to the medical treatment of the patient. The system and the method of the invention provide information that the medications are taken correctly and at right time. Secondly, the system and the method assists the patient in other care events like measuring e.g. blood pressure or blood glucose level and may provide information relating to the measuring results, which is useful for determining effects of the medications. In addition, the system and the method provide information relating to the patient's condition, possible side-effects and feelings by asking direct feedback questions from the patient. Thus, the medication of the patient can be easily optimized or changed, and the caregivers can quickly react to a change in the patient's conditions.

The exemplary embodiments of the invention presented in this text are not interpreted to pose limitations to the applicability of the appended claims. The verb "to comprise" is used in this text as an open limitation that does not exclude the existence of also unrecited features. The features recited in the dependent claims are mutually freely combinable unless otherwise explicitly stated.

The exemplary embodiments presented in this text and their advantages relate by applicable parts to the method as well as the system according to the invention, even though this is not always separately mentioned.

### BRIEF DESCRIPTION OF THE DRAWINGS

The novel features which are considered as characteristic of the invention are set forth in particular in the appended claims. The invention itself, however, both as to its construction and its method of operation, together with additional objects and advantages thereof, will be best understood from the following description of specific embodiments when read in connection with the accompanying drawings.
- Fig. 1: illustrates a medication administration system according to an embodiment of the invention, and
- Fig. 2: illustrates a schematic diagram of a method according to an embodiment of the invention.

### DETAILED DESCRIPTION OF THE DRAWINGS

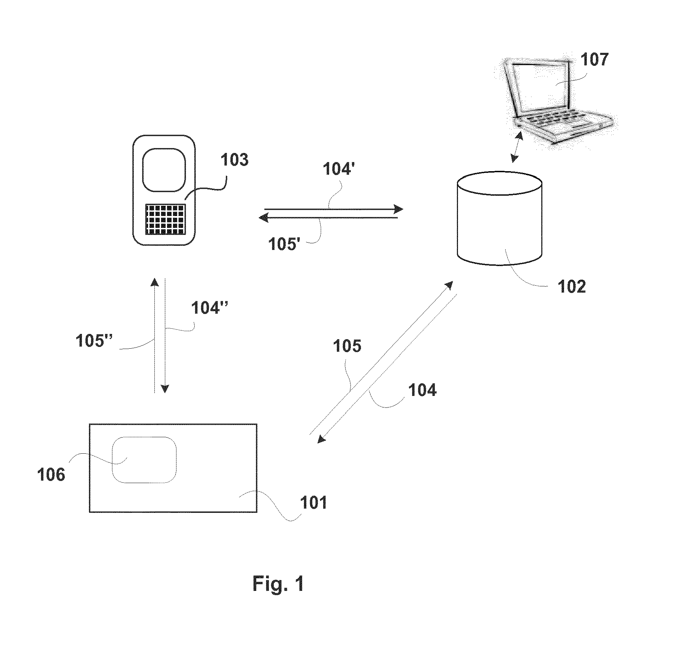

Figure 1 illustrates a medication administration system according to an embodiment of the invention. The system comprises a medication dispenser 101 which includes medication packages to be taken, and a server 102 which is arranged to be communicatively connected with the medication dispenser. Instructive messages 104, 104', 104" can be sent to the dispenser 101 via the server 102 or directly from a predefined mobile device 103. To the server 102 messages are sent through a web user interface from a terminal 107 communicatively connected to the server or from a mobile device 103. Typically, the dispenser 101 comprises a user interface 106, such as a touch screen, in order that the messages 104, 104" may be displayed to the user of the dispenser.

Messages 105, 105', 105", such as feedback or a question relating to the medication or other care events can be sent from the dispenser 101 to the server 102, via the server 102 to the mobile device 103 or directly to the predefined mobile device 103. Messages can be also reviewed through the web user interface from the terminal 107 communicatively connected to the server 102.

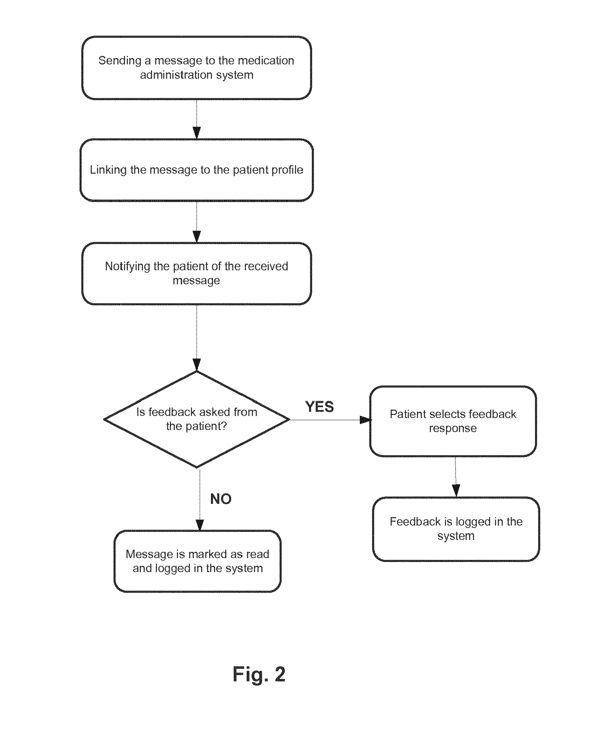

Figure 2 illustrates a schematic diagram of a method according to an embodiment of the invention. The method is meant for remote messaging for assisting the patient, i.e. the user of the dispenser, in medication and other care events. The presented method comprises sending a message to the system and linking the message to the patient profile available in the system. The patient is notified of the received message at a predefined time depending on the linkage of the message. The message notified to the patient may comprise a feedback question, which question the patient can answer by selecting a desired answer alternative from the alternatives displayed to the patient. The selected feedback answer is stored in the system. If the message does not comprise a feedback question, the message is marked as read and logged as read message in the system after it has been displayed to the patient.

Only advantageous exemplary embodiments of the invention are described in the figures. It is clear to a person skilled in the art that the invention is not restricted only to the examples presented above, but the invention may vary within the limits of the claims presented hereafter. Some possible embodiments of the invention are described in the dependent claims, and they are not to be considered to restrict the scope of protection of the invention as such.

## Claims

1. A method for a medication administration system, which system comprises a medication dispenser, which includes medication packages containing medications to be taken at predefined taking times, and a medication administration server which is arranged to be communicatively connected with the medication dispenser over a communications network,
**characterised in that** the method comprises:
- providing the medication dispenser with a patient profile, which profile comprises identification information of the patient and information related to medications of the patient and taking periods for the medication packages,
- providing the system with communication means, which are configured to receive and/or transmit a message, and
the method further comprises
- sending a message to the system for assisting and instructing the patient remotely in medication or other care events, linking the message to a predefined information in the patient profile, and notifying the patient of the received message,
and/or
- supplying a message to the system from the medication dispenser.

2. The method according to claim 1**, characterised in that** the step of sending a message to the system comprises:
- sending the message to the medication dispenser by communication means via the server or directly from a predefined terminal.

3. The method according to any of the preceding claims, **characterised in that** the step of notifying the patient of the received message comprises:
- reading the information of the medication package to be dispensed from the dispenser,
- comparing the information read from the package to the information of the patient profile stored in the system,
- identifying a message linked to the information in the patient profile and corresponding to the information read from the package, and
- retrieving the received message from the memory of the server or the dispenser.

4. The method according to any of the preceding claims, **characterised in that** the message is notified to the patient at a predefined time or directly when it has been sent to the system.

5. The method according to any of the preceding claims, **characterised in that** the method comprises an audio and/or visual notification of the received message.

6. The method according to any of the preceding claims, **characterised in that** the method comprises:
- displaying the message on the user interface of the medication dispenser.

7. The method according to any of the preceding claims, **characterised in that** the step of supplying a message to the system by using the medication dispenser further comprises:
- sending a message from the medication dispenser over a communications network to the server or directly to a predefined terminal.

8. The method according to any of the preceding claims, **characterised in that** the method comprises:
- requesting a feedback from the patient and transmitting the answer to the server or to a predefined terminal.

9. The method according to claim 8, **characterised in that** the message with feedback request comprises question and reply alternatives relating to the patient's physical or mental health or welfare.

10. The method according to any of the preceding claims, **characterised in that** the method further comprises:
- storing the message in a memory of the dispenser or in the server of the system.

11. The method according to any of the preceding claims, **characterised in that** the method comprises:
- opening a voice or video call between the patient and a predefined recipient.

12. A medication administration system, comprising:
- a medication dispenser, which includes medication packages containing medications to be taken at predefined taking times, and
- a medication administration server which is arranged to be communicatively connected with the medication dispenser over a communications network,
**characterised in that** the system further comprises
- means for receiving a patient profile, which the profile comprises identification information of the patient and information related to medications of the patient and taking periods for the medication packages,
- means for receiving a message for assisting and instructing the patient remotely in medication or other care events, and means for linking the message to a predefined information in the patient profile,
- means for notifying the patient of the received message, and
- means for supplying a message to the system from the medication dispenser.

13. The system according to claim 12, **characterised in that** the medication dispenser comprises a user interface, preferably a touch screen.

14. The system according to claim 12 or 13, **characterised in that** the system further comprises means for sending a message from the dispenser to the server or to a predefined terminal.

15. The system according to any of the preceding claims 12 to 14, **characterised in that** the medication dispenser comprises a microphone and speaker for a voice call, and/or a camera for a video call.
